# EUROPEAN PATENT SPECIFICATION

(11) **EP 3 863 544 B1**
(45) Date of publication and mention of the grant of the patent: **18.01.2023**
(21) Application number: 19798393.5
(22) Date of filing: 04.10.2019
(51) Int. Cl.: A61B 17/70

(54) **IMPROVED INTERLAMINAR-TYPE INTERVERTEBRAL SUPPORT DEVICE**
VERBESSERTE INTERLAMINARE ZWISCHENWIRBELSTÜTZVORRICHTUNG
DISPOSITIF DE SUPPORT INTERVERTÉBRAL DE TYPE INTERLAMINAIRE AMÉLIORÉ

(30) Priority: 04.10.2018 IT 201800009150
(43) Date of publication of application: 18.08.2021
(73) Proprietor: G & G S.R.L., 50131 Firenze (IT); Spineas Inversiones, Societad Limitada, 38660 Arona S.C. Tenerife (ES)
(72) Inventor: PETRINI, Piero, 06012 Citta' Di Castello (IT); GUIZZARDI, Giancarlo, 50141 Firenze (IT)
(74) Representative: ABM Agenzia Brevetti & Marchi
(86) International application number: PCT/IB2019/058447
(87) International publication number: WO 2020/070697

(56) References cited:
- WO-A1-2005/044118
- WO-A2-2008/056237
- US-A1- 2005 261 768
- US-A1- 2011 029 021
- US-A1- 2015 282 944

## Description

### Field of the invention

The present invention relates to an interlaminar-type intervertebral support device, i.e. a device comprising a portion configured to be inserted between the laminae of two adjacent vertebrae. This device is an improvement of the device described in WO 2005/044118.

Like the device of WO 2005/044118, the new device is useful for treating a degenerative condition of an intervertebral disc, also in an incipient state, by more evenly distributing the extra-loads due to the degenerative condition on the adjacent vertebrae, while preserving the relative articular mobility of the two vertebrae.

### Background of the invention

WO 2005/044118 discloses an interlaminar support 80, as shown in Figs. 1-3, comprising a front support portion 81 and a rear stabilization portion 82. Support portion 81 is configured to be placed between the laminae 15,16 of two adjacent vertebrae 10',10", in order to restore an intervertebral anatomical gap between vertebrae 10',10". Stabilization portion 82 has a substantially prismatic shape and comprises protruding parts 88 and/or 93 extending from support portion 81 at a position more backward than support portion 81. Protruding parts 88 and/or 93 are configured to abut against the rear surfaces 19 of laminae 16, and serve to assist the surgeon when he/she positions interlaminar support 80 with support portion 81 at a distance not shorter than a safety distance from the spinal cord 14, so as to prevent interlaminar support 80 from shifting towards spinal cord 14 once the device has been implanted. This way, device 80 cannot cause injury to the spinal cord while implanting the device and subsequently.

Stabilization portion 82 is also useful to prevent the interlaminar support 80 from:
- moving backwards with respect to the spine, i.e. from leaving its implant site;
- rotating about a horizontal transverse axis.

This object is achieved thanks to the friction forces acting between the spinous processes 17,18 and the upper and lower surfaces 84,85, respectively, of stabilization portion 82. As well known, this friction is present only if normal forces are applied between device surfaces 84,85 and the surfaces of spinous processes 17,18, i.e. only if compression forces are applied by spinous processes 17,18 to stabilization portion 82, which correspond to reaction spacing-apart forces that the stabilization portion 82 applies to spinous processes 17,18. All this has some drawbacks:
- firstly, this stabilization by friction could be unsuccessful, since it is affected by the surface conditions of the spinous processes and by their shape, as well as by the elastic properties of interlaminar support 80 and, therefore, the intensity of the stabilization forces contrasting the device expulsion cannot always be accurately determined. In particular, if an even minimum residual device expulsion risk exists, disadvantageously, a new surgical operation is required involving once again the supraspinous ligament 12 behind the implant site, which must be once more opened and then sutured. This would obviously cause discomfort and even possible complications to the patient, besides increasing the overall cost of the treatment. Moreover, a preparation of the surfaces of the spinous processes may be required in order to enhance the friction with the device;
- secondly, in order to provide a friction strong enough to stabilize the device, upper and lower surfaces 84,85 and, therefore, stabilization portion 82 must be somewhat large, which complicates positioning interlaminar support 80 in such a small and hardly accessible implant site;
- thirdly, the reaction forces acting on the spinous processes cannot always be tolerated by the latter, in particular if the spinous processes are weakened due to such conditions as osteoporosis and the like, in which cases injuries to the spinous processes can occur.

Moreover, the shape of stabilization portion 82 makes device 80 not well suited to some patient's anatomical variants.

Moreover, the shape of the stabilization portion 82 makes device 80 not well suited for vertebrae at the uppermost spinal levels, in particular for the cervical vertebrae.

Moreover, support surfaces 83',83" of support portion 81 have a linear edge, while laminae 15,16 have a curved profile, since they are portions of the vertebral arch. Therefore, device 80 mainly supports the central part of the vertebral arch of each vertebra 10',10", i.e. the part corresponding to laminae 15,16 next to respective spinous processes 17,18, while the part farther from the spinous processes are not supported by support portion 81, and do not contribute to more evenly distribute the intervertebral forces. On the other hand, as described above, support portion 81 cannot be arranged beyond a certain safety limit position, in order to prevent spinal cord 14 from being injured. The safety limit position is related to the position of the yellow ligament, not shown, which connects the inner surfaces of laminae 15,16 in the rear portion of respective vertebral archs.

On the other hand, FR 2 816 197 discloses an interspinous-type intervertebral device made of a resilient and relatively soft material, in which two pairs of arms extend from opposite sides of a central portion, typically like the four legs of a letter H with respect to the central portion thereof. Each pair of arms defines a space arranged to receive a spinous process of the vertebrae above and below the implant site, respectively. This device is configured to come into contact with the vertebrae only at the spinous processes. Two arms belonging to one pair and to the other pair of arms, respectively, which protrude in opposite directions from the same side of the central portion, are configured to be resiliently-rotated in an opposite direction with respect to the other arm of the same pair. This way, the device can be brought to a minimum-transversal size deformed configuration. A retaining means is also provided to retain the arms in this deformed configuration, so that the device can be inserted through a minimum-size back or posterolateral access. The retaining means is removed once the positioning has been carried out, so that the arms recover their "H"-rest configuration, and embrace the spinous processes.

US 2011/0029021 A1 describes an intervertebral spacer configured for introduction between relevant bone structures of two adjacent vertebrae, in order to maintain a predetermined intervertebral distance, said spacer comprising a plurality of plates, each having a central base portion and a plurality of flanges, typically four X-arranged flanges, which radially extend from the base portion, so as to be resiliently bent with respect to the plane where the base portion lies, the plates arranged to be moved towards / away from each other along a central axis, and to selectively turn from a compressed configuration, allowing the spacer to be implanted through a minimally invasive access, to an expanded configuration, to be set once the device has been positioned / and vice-versa. US 2011/0029021 A1 only exemplifies the case of an interspinous-type spacer, in which the X-shaped stack of flanges is arranged between the spinous processes of two adjacent vertebrae, so as to receive the two processes in respective upper and lower spaces, opposite to each other, defined between the upper arms and between the lower arms of the X-shape. Once this device has been positioned and brought to the extended configuration, the device has a substantially uniform height starting from a proximal end portion, facing the spinal channel, and going towards the distal end portion, oriented backwards with respect to the patient. Therefore, once such a device has been positioned and brought to the extended configuration, it locally imposes a constraint to the extension movement of the spine, which makes it impossible to correctly restore the physiologic lordosis of the spine portion including the two adjacent vertebrae.

US 2015/0282944 A1 describes a vertebral fusion system comprising a plurality of fusion devices configured to be positioned between corresponding vertebral portions such as vertebral processes and the vertebral bodies of the adjacent vertebrae. Among the above fusion devices, an interspinous fusion device is provided that is configured to be positioned between the spinous processes of the two vertebrae. These fusion devices comprise a container with open end portions and a poorly plastically compressible granular osteosynthesis material arranged within the container and adapted to integrate with the vertebral portions between which the fusion device is arranged. In an exemplary embodiment, the interspinous fusion device comprises a front (distal) portion configured to be introduced between the laminae.

### Summary of the invention

It is therefore the object of the present invention to provide an interlaminar-type intervertebral support that is stabilized in the implant site without substantially requiring the cooperation of friction forces exerted between itself and the spinous processes for the primary stabilization, or in any case to provide an interlaminar-type intervertebral support that only slightly stresses the spinous processes, in comparison with the prior art devices.

It is the object of the invention to provide such an intervertebral interlaminar-type support device that can easily fit any patient's anatomical variants.

Moreover, it is the object of the present invention to provide such a device that can be implanted at any spinal level, in particular at a cervical level.

It is also a feature of the invention to provide an interlaminar-type intervertebral support device that provides a wider support surface for the laminae than the above-mentioned prior art devices, at the rear portion of the vertebral arch of each vertebra, so as to more evenly distribute the intervertebral forces on a wider surface, in comparison with such prior art devices.

It is also a feature of the invention to provide such an intervertebral support that can be implanted by a minimally invasive technique, in particular by a percutaneous procedure.

This object is achieved by an interlaminar-type intervertebral support device as defined by independent claim 1. Particular exemplary embodiments of the invention are defined by the dependent claims. According to the invention, an interlaminar-type intervertebral support device for implant between a lower vertebra and an upper vertebra, said vertebrae having respective pairs of right and left laminae and respective spinous processes, comprises:
- a proximal support portion configured to be inserted between the pairs of right and left laminae and providing lower and upper support surfaces configured to abut against the pairs of laminae of the lower and upper vertebrae, respectively;
- a distal stabilization portion adjacent to the proximal support portion along a longitudinal axis of the device;
wherein the distal stabilization portion comprises a tail element extending from a side opposite to the proximal support portion and configured to abut against the root portion of the spinous process of the lower vertebra,
wherein the distal stabilization portion comprises an engagement section for engaging with the laminae, said engagement section comprising:
- a lower pair of right and left radial ramifications extending along respective lower radial axes and configured to abut against external faces of the right and left laminae, respectively, of the lower vertebra, when the proximal support portion is inserted between the pairs of right and left laminae;
- an upper pair of right and left radial ramifications extending along respective upper radial axes and configured to abut against external faces of the right and left laminae, respectively, of the upper vertebra, when the proximal support portion is inserted between the pairs of right and left laminae,
wherein said central core has, between said upper pair of right and left radial ramifications, an upper face having a downward slope with respect to said support surfaces, starting from a distal side of said stabilization portion, adjacent to said interlaminar proximal support portion, and going towards a proximal end side of said device.

This way, the two upper and lower pairs of right and left radial ramifications are arranged onto the laminae in a radially distributed position starting from their own root portions, next to the distal stabilization portion, up to their own free end portions, providing a wide support to the laminae, and preventing the laminae and the device to exert concentrated forces to each other, i.e. forces applied on respective small surfaces.

Moreover, thanks to the radial ramifications, the primary stabilization process of the device does not involve extended surface portions of the spinous processes, unlike the cited prior art device, as previously detailed.

Moreover, the tail element assists the primary stabilization of the device especially in that it prevents the device from rotating about both the longitudinal axis and a transversal axis perpendicular to the longitudinal axis.

Moreover, the slope of the upper face of the central core of the stabilization portion allows any physiological extension movement of the spine portion including the two adjacent vertebrae, once the device has been arranged in the implant site. This way, the extension movement of the spine are not appreciably hindered, unlike the device disclosed in US 2011/0029021 A1. Therefore, by implanting the device according to the invention, it is possible to restore the physiologic lordosis of the spine portion including the two adjacent vertebrae.

Advantageously, the lower radial axes are at a predetermined lower inclination angle with respect to a sagittal midplane, said lower inclination angle set between 60° and 80°, in particular set between 65° and 75°, more in particular, said lower inclination angle is about 70°, while the upper radial axes are at a predetermined upper inclination angle with respect to the sagittal midplane, said upper inclination angle set between 45° and 60°, in particular set between 50° and 55°, more in particular, said upper inclination angle is about 52°. Those angles allow an ideal anatomical positioning of the ramifications.

According to the present invention, the right and left radial ramifications of at least one pair selected between the lower pair and the upper pair are configured to resiliently perform respective rotations about the longitudinal axis, from a rest position to a rotated position, so as to bring the engagement section from a rest configuration to a resiliently-rotated configuration, and vice-versa. Preferably, both the radial ramifications of the lower pair and the radial ramifications of the upper pair are configured to rotate resiliently.

This way, since at least one pair of the two upper and lower pairs of right and left radial ramifications, or both pairs, is/are resiliently compliant so as to rotate about their own root, i.e. about the longitudinal axis, it/they can fit the actual shape of the laminae, which can be different from an anatomical model that is determined from outside the body and that does not necessarily correspond to that actual shape. Moreover, the deformability of the radial ramifications allows them to follow the relative anatomical motions of the joints, while bearing the loads at the laminae.

A further advantage is that, since the ramifications are resiliently compliant, the support device can be introduced into a patient's body through a minimum-size opening. In particular, during an implant procedure of this support device, the latter can be inserted within a cannula. The cannula can be in turn introduced into a minimum-size surgical access, typically into a posterolateral access, and then positioned with an outlet end thereof at the implant site of the device.

A push means can be provided within the cannula, said push means configured to maintain the support device at the implant site while causing the cannula to slide back in order to withdraw it from the patient's body, until the support device is released by the cannula itself. Then, the containing action of the cannula is no longer present, so the device resiliently recover its initial configuration, in which the lower and upper radial ramifications have a shape in the space suitable for optimally abutting against the external surfaces of the lower and upper laminae, respectively. Therefore, due to the resilient deformability of the radial ramifications, an interlaminar device is provided that maintains the advantages described above, and that can be also implanted in a minimally invasive way, which drastically reduces discomfort to the patient, along with time and costs of the treatment.

In particular, when the right and left radial ramifications are in the rest configuration, the lower radial axes and the upper radial axes are at said lower and upper inclination angles, respectively, with respect to the sagittal plane. This way, the ramifications arranged within the above angle ranges will accurately follow the anatomical conformation of the external faces of the laminae.

Preferably, the right and left radial ramifications configured to rotate resiliently have at least a root portion that is made of a resiliently deformable material. This way, the deformability of the support device is obtained without introducing mechanical links between the radial ramifications and the body of the device, which simplifies the construction and makes the device more reliable over time.

Advantageously, the lower radial ramifications have a convex proximal face and the upper radial ramifications have a concave proximal face. This improves the contact between the radial ramifications and the external surfaces of the laminae, which assists the primary stabilization of the device.

In another advantageous exemplary embodiment, the support surfaces of the proximal support portion have side parts that extend more forward in a proximal direction than the respective central parts. This way, the support surface for the laminae provided by the interlaminar support portion is increased, which reduces the stress exerted on the device and improves the support performance. In fact, as anticipated, the laminae are a part of the vertebral arch and have therefore a curvature in a transversal plane of the spine. Therefore, the side parts, extending more forward than the central part, can reach the farthermost regions of the laminae, with respect to the sagittal plane of the spine, whereas the central part, extending less forwards, will be placed at a safety distance from the dura mater.

In particular, the tail element has a protruding length, with respect to the lower right and left radial ramifications, that is shorter than 10 mm, preferably shorter than 8 mm, even more preferably shorter than 6 mm.

Therefore, the tail element preferably comes into contact only with the spinous process of the lower vertebra. This way, no compression force is exerted between the tail element and the spinous processes of the lower and upper vertebrae, or only forces are exerted that are too weak to be dangerous for the spinous processes, even in the case of spinous processes that are weakened due to such patient's conditions as osteoporosis or the like.

Advantageously, the tail element has a lowered upper surface portion forming a recess configured in such a way to allow a movement of the spinous process of the upper vertebra. This way, the spinous processes are prevented from compressing the device, thus preventing such related risks as kyphotization and overstressing of the spinous processes.

Preferably, the distance between free end portions of right and left radial ramifications of the upper pair or of the lower pair of radial ramifications is set between 25 and 35 mm, in particular it is set between 27 mm and 33 mm.

Preferably, the distance between the free end portions of the right radial ramifications and between the free end portions of the left radial ramifications is set between 14 and 22 mm, in particular is set between 16 mm and 20 mm.

Preferably, the protruding length of the upper support surface of the proximal support portion from the proximal face of the upper radial ramifications is set between 4 mm and 6 mm.

Preferably, the distance between the lower support surface and the upper support surface is set between 4 mm and 14 mm, in particular is set between 5 and 12 mm.

In a device that has at least one pair of radial ramifications configured to rotate resiliently, said one pair selected among the lower pair and the upper pair, the above-mentioned distance between the free end portions of the right and of the left radial ramifications is a distance in the rest configuration, while, in the resiliently-rotated configuration, this distance between the free end portions is shorter than 18 mm, in particular it is shorter than 15 mm. Such size is suitable for insertion into a surgical cannula, when implanting the support device by a minimally invasive technique.

### Brief description of the drawings

The invention will be now shown with the description of its exemplary embodiments, exemplifying but not limitative, with reference to the attached drawings, in which:

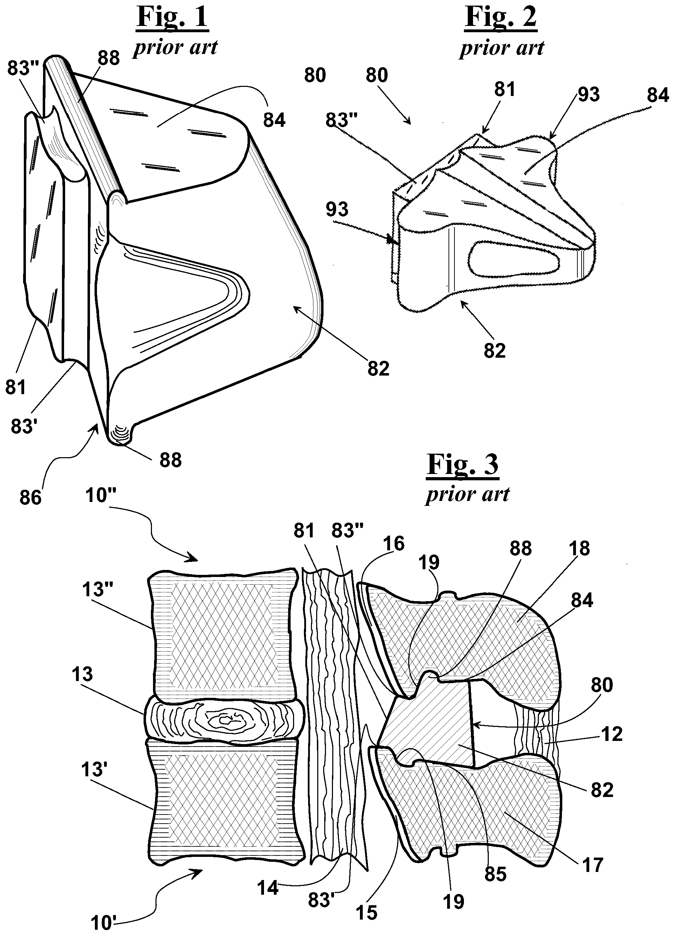

- Figs. 1 and 2 are perspective views of two interlaminar support devices according to corresponding exemplary embodiments of a prior art device;
- Fig. 3 is a diagrammatical cross sectional view of the device of Fig. 1 implanted between two vertebrae;
- Figs. 4 and 7 are perspective views of a device according to the invention as seen from two different front and rear points of view;
- Fig. 4A is a longitudinal sectional view of the device of Figs. 4 and 7, taken along the mid-sagittal plane of the device;
- Figs. 5 and 6 are front elevation views of an interlaminar support device according to an advantageous exemplary embodiment of the invention in a spread-apart rest configuration and in a narrowed forced configuration, respectively;
- Fig. 8 is a side elevation view of a device according to an exemplary embodiment of the invention, in which the proximal surfaces of the radial ramifications have convex and concave parts;
- Fig. 9 is a top plan view of a support device according to another advantageous exemplary embodiment of the invention, in which the support surfaces for the laminae have an extension changing between the center and the periphery;
- Fig. 10 is a front elevation view of one of the devices of Figs. 4-12, in which some dimension are indicated;
- Fig. 11 is a front elevation view of the device of Fig. 7, in which some dimensions are indicated;
- Fig. 12 is a front elevation view of the device of Fig. 8, in which some dimensions are indicated;
- Figs. 13-15 are a diagrammatical cross sectional view, a rear view and a postero-lateral view of a lumbar spine portion, in which the support device according to an exemplary embodiment of the invention located between two adjacent vertebrae.

### Description of a preferred exemplary embodiment

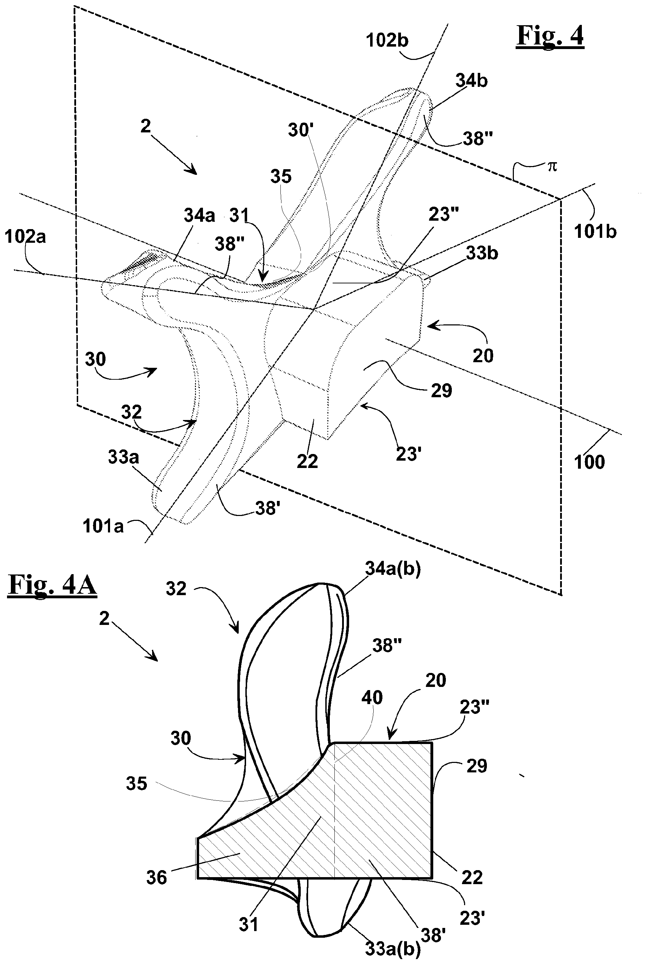

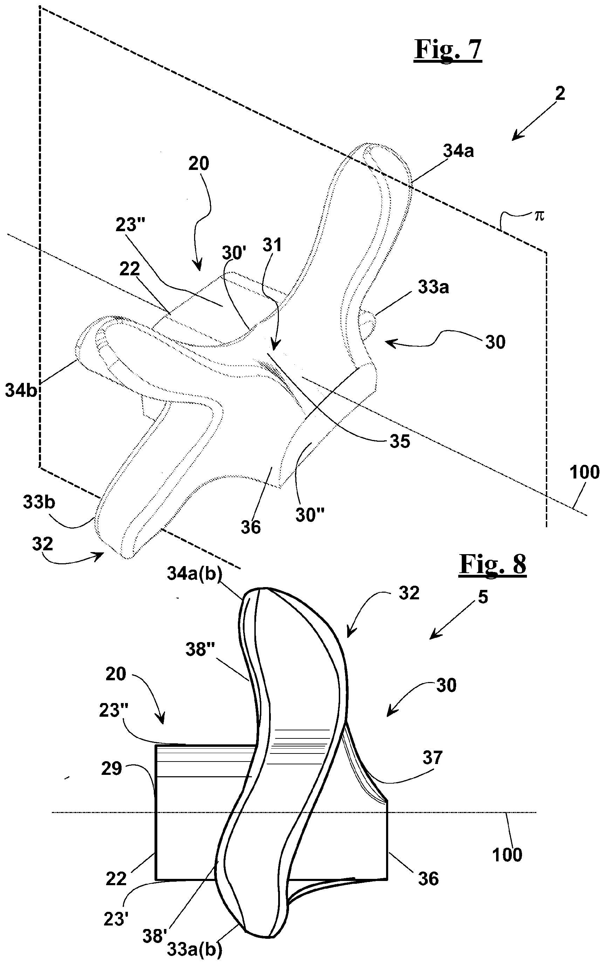

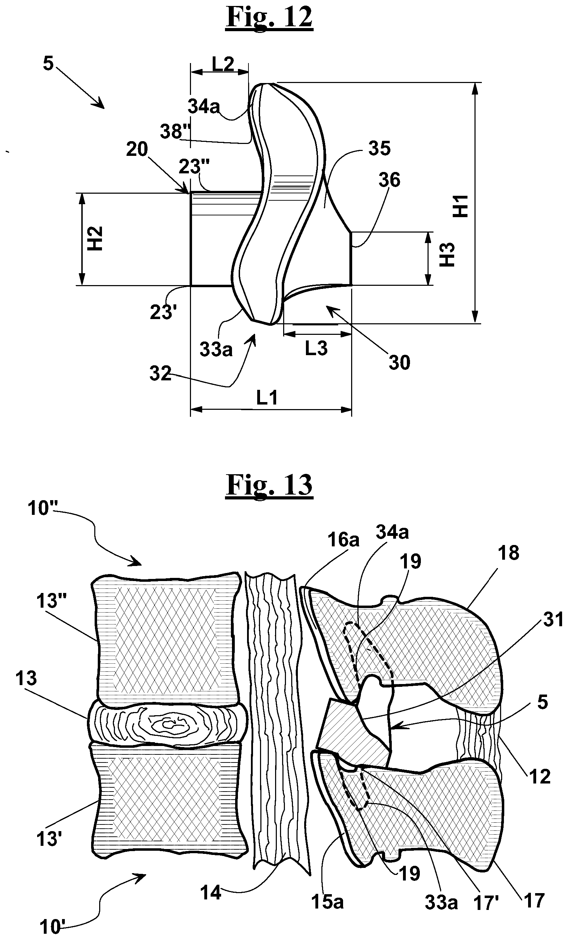

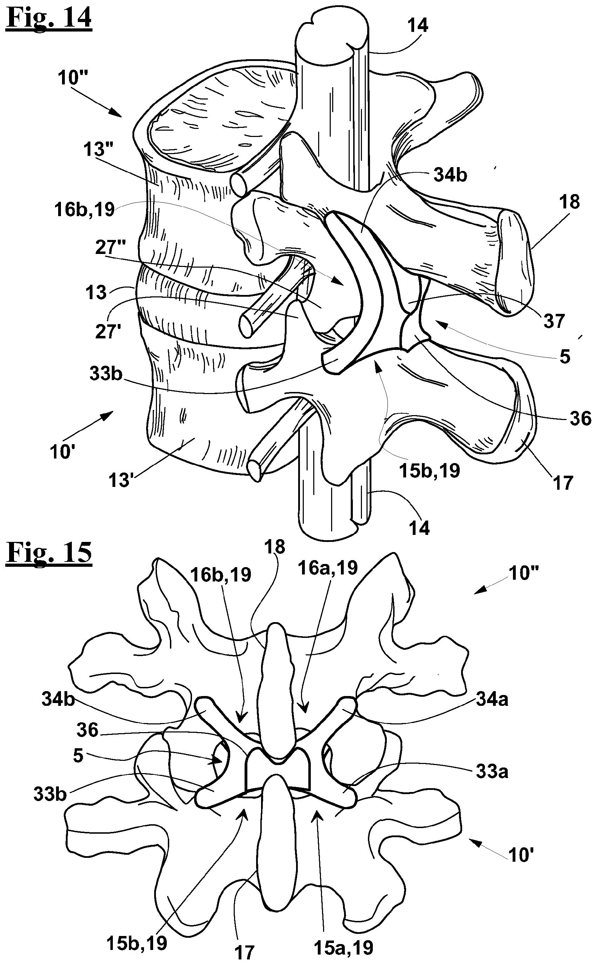

With reference to Figs. 4, 4A and 7, an interlaminar-type intervertebral support device 2 is described, according to a possible exemplary embodiment, configured to be implanted between a lower vertebra 10' and an upper vertebra 10" of a subject's spine portion, as indicated in Figs. 13-15, which show anatomical details among which vertebral bodies 13',13" of two lower and upper adjacent vertebrae 10',10", respectively, an intervertebral disc 13 between vertebral bodies 13',13", spinous processes 17,18 of lower and upper vertebrae 10',10", respectively, pairs of laminae 15a,b and 16a,b of lower and upper vertebrae 10',10", respectively, and articular processes 27',27" of lower and upper vertebrae 10',10", respectively. In Fig. 13, the cortical portion and the trabecular portion of the cross-sectionally shown bones are represented as differently hatched areas.

Still with reference to Figs. 4, 4A and 7, support device 2 comprises an interlaminar proximal support portion 20 configured to be inserted between pair of laminae 15a,b of lower vertebra 10' and pair of laminae 16a,b of upper vertebra 10'. To this purpose, interlaminar proximal support portion 20 has lower and upper support surfaces 23',23" configured for abutment against pair of laminae 15a,b of lower vertebra 10' and of pair of laminae 16a,b of upper vertebra 10', respectively.

Support device 2 also comprises a distal stabilization portion 30 adjacent to interlaminar proximal support portion 20 along a longitudinal axis 100 of device 2. According to the invention, distal stabilization portion 30 has an engagement section 32 for engaging with laminae 15a,b and 16a,b, comprising a lower pair of right and left radial ramifications 33a,b that extend starting from a central core 31 of stabilization portion 30 along respective lower radial axes 101a,b, and an upper pair of right and left radial ramifications 34a,b that extend from central core 31 along respective upper radial axes 102a,b.

Lower and upper radial ramifications 33a,b and 34a,b are configured to abut against external faces 19 of laminae 15a,b and 16a,b of lower and upper vertebrae 10',10", respectively, when interlaminar proximal support portion 20 is inserted between the pairs of right and left laminae 15a,b and 16a,b.

Central core 31 of stabilization portion 30 has an upper portion between upper radial ramifications 34a,b with a surface 35 at an angle with respect to support surfaces 23',23" of interlaminar proximal support portion 20, more in detail, surface 35 has a downward slope from a distal side 30' of stabilization portion 30, adjacent to interlaminar proximal support portion 20, towards a proximal end side 30" of device 10. Therefore, in a longitudinal cross section taken along a mid-sagittal plane π passing through longitudinal axis 100, as shown in Figs. 4A and 13, central core 31 has a substantially trapezoidal shape 40, or even of a triangular shape, apart from a preferably curvilinear edge, said trapezoidal shape having the longer base in a distal position and the shorter base or the vertex opposite to the longer base in a proximal position. This slope allows any physiological extension movement of the spine portion including two adjacent vertebrae 10' and 10", once the device has been located in its implant site, i.e. it does not limit the extension movement of the spine, unlike the device described in US 2011/0029021 A1, and allows therefore to correctly restore the physiologic lordosis of the spine portion including two adjacent vertebrae 10',10".

Right and left radial ramifications 33a,34a and 33b,34b are advantageously symmetrical with respect to sagittal plane π of the device, passing through longitudinal axis 100.

Interlaminar proximal support portion 20 is a projection 22 of distal stabilization portion 30, and preferably has the shape of a parallelepiped or, in any case, of a prism preferably with beveled edges, said prism having two parallel faces providing lower and upper support surfaces 23',23", and an end base 29 opposite to distal stabilization portion 30.

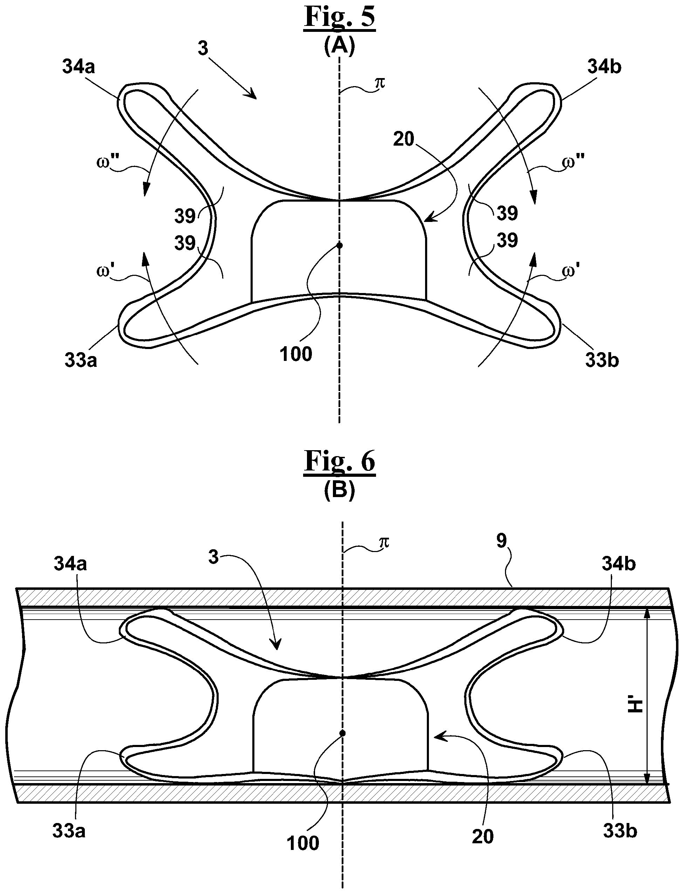

With reference to Figs. 5 and 6, an intervertebral support device 3 is described according to an advantageous exemplary embodiment of the invention, in which right and left radial ramifications 33a,34a and 33b,34b of the lower pair and/or of the upper pair are configured to resiliently perform rotations ω' and/or ω" about longitudinal axis 100 of support device 3, starting from a rest configuration A (Fig. 5), towards / away from the corresponding radial ramifications of the other pair of radial ramifications, until it reaches a predetermined resiliently-rotated configuration B (Fig. 6).

Support device 3, according to this exemplary embodiment, is well suited for a mini-invasive implant procedure. More in detail, as shown in Fig. 6, support device 3 can be inserted into a cannula 9 that can in turn be introduced through a typically posterolateral minimum-size surgical access, not shown, and can preferably be arranged with an own end at the implant site.

Preferably, radial ramifications 33a,33b and 34a,34b of both lower and upper pairs are configured to resiliently perform respective rotations ω₁, ω₂, as shown in Fig. 6.

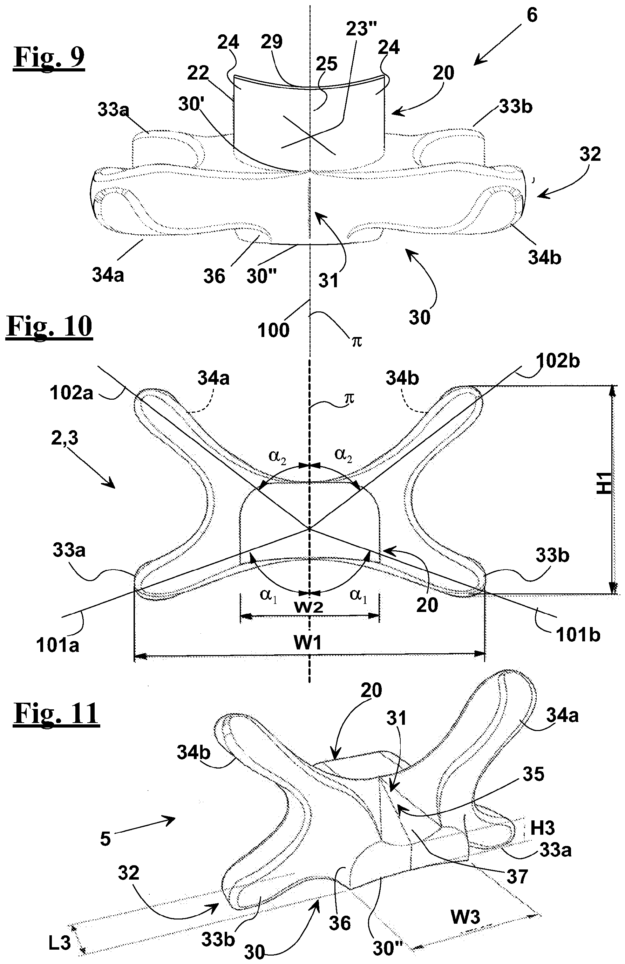

As shown in Fig. 10, lower radial axes 101a,b, along which respective lower radial ramifications 33a,b extend, are at a lower inclination angle α₁, preferably set between 60° and 80°, with respect to sagittal midplane π of the device. More preferably, lower inclination angle α₁ is set between 65° and 75°, and even more preferably lower inclination angle α₁ is about 70°.

Similarly, upper radial axes 102a,b, along which respective upper radial ramifications 34a,b extend, are at an upper inclination angle α₂, preferably set between 45° and 60°, with respect to sagittal midplane π. More preferably, upper inclination angle α₂ is set between 50° and 55°, and even more preferably upper inclination angle α₂ is about 52°.

In case of support device 3, in which radial ramifications 33a,b and/or 34a,b of at least one pair can rotate resiliently, these values of inclination angles α₁ and α₂ correspond to the slopes of radial axes 101a,b and 102a,b in the rest position A (Fig. 5).

The resilient rotation of radial ramifications 33a/b and/or 34 a/b can be variously performed. For instance, a resiliently deformable material can be used to make at least a root portion 39, i.e. a portion next to longitudinal axis 100, of each resiliently rotatable radial ramification 33a/b and/or 34a/b. Preferably, in this case, the whole resiliently rotatable radial ramification 33a/b and/or 34a/b is made of such a resiliently deformable material, and even more preferably the whole support device 3 is made of such a resiliently deformable material.

According to an alternative exemplary embodiment, not shown, the resilient rotation of radial ramifications 33a/b and/or 34a/b can be manufactured as hinge elements arranged at the root portion of each resiliently rotatable radial ramification 33a/b and/or 34a/b, said hinge elements associated with a torsion spring.

The skilled person will be able to easily select the resiliently deformable material among the materials that comply with the requirements of biocompatibility and mechanical strength for this specific application, in any case this resilient material is preferably selected among silicone resins. The skilled person will be able to easily select a hinge element to provide a joint between the radial ramifications and the rest of device 3.

With reference to Fig. 8, an interlaminar-type intervertebral support 5 is described according to another exemplary embodiment of the invention, in which lower and upper radial ramifications 33a,b and 34a,b have convex and concave proximal faces 38',38", respectively. In particular, this can be obtained as shown in Fig. 8, by a curved conformation of radial ramifications 33a,b and 34a,b, as observed from a lateral point of view.

With reference now to Fig. 9, an interlaminar-type intervertebral support 6 is described according to a further particularly advantageous exemplary embodiment of the invention, in which support surfaces 23',23" of interlaminar proximal support portion 20 have side parts 24 extending more forward in a proximal direction with respect to corresponding central parts 25. In particular, interlaminar proximal support portion 20 has a concave front or proximal profile 29 with a curvature selected taking into account the curvature of laminae 15a,b and 16a,b (portion of the vertebral arch).

As shown in Fig. 7, distal stabilization portion 30 of support device 2, according to the invention, preferably comprises, besides engagement section 32, a tail element 36 also aligned with interlaminar support portion 20 and arranged opposite to the latter, with respect to radial ramifications 33a,b and 34a,b. This tail element is configured to abut against a root portion 17' of spinous process 17 of lower vertebra 10'. To this purpose, tail portion 36 has a protruding length L3 (Figs. 11 and 12), with respect to the right and left radial ramifications, shorter than 10 mm, in particular shorter than 8 mm, even more in particular, shorter than 6 mm.

In particular, in an intervertebral support device 5 shown in Fig. 11 (and also in Figs. 8 and 12), according to a modification of this exemplary embodiment, tail element 36 has a lowered upper surface portion 37 so as to form an anatomical recess configured to contain spinous process 18 of upper vertebra 10'.

Figs. 10, 11 and 12 show the sizes of devices according to some exemplary embodiments the invention.

Preferably, a distance W1 (Fig. 10) between the free end portions of lower radial ramifications 33a,33b and between the free end portions of upper radial ramifications 34a,34b is set between 25 and 35 mm, in particular it is set between 27 mm and 33 mm.

Preferably, a distance H1 (Figs. 10 and 12) between the free end portions of right radial ramifications 33a,34a and between the free end portions of left radial ramifications 33b,34b is set between 14 and 22 mm, in particular it is set between 16 mm and 20 mm.

Preferably, a protruding length L2 (Fig. 12) of upper support surface 23" of interlaminar proximal support portion 20 by a proximal face 38", of support to external face 19 of one of the laminae 16a,b of upper vertebra 10', is set between 4 mm and 6 mm.

Preferably, the distance H2 (Fig. 12) between the lower support surface 23' and upper support surface 23" of interlaminar proximal support portion 20 is set between 4 mm and 14 mm, in particular between 5 and 12 mm.

In case of the support device as device 3 of Figs. 5 and 6, where radial ramifications 33a,b and/or 34a,b of at least one pair can rotate resiliently, the values of the distance H1 above indicated refer to a rest position A, whereas in the resiliently-rotated configuration B the corresponding distance H' (Fig. 6) is shorter than 18 mm, preferably less than 15 mm.

## Claims

1. An interlaminar-type intervertebral support device (2,3,5,6) configured to be implanted between a lower vertebra (10') and an upper vertebra (10"), said lower and upper vertebrae having respective pairs of right and left laminae (15a,b; 16a,b) and respective spinous processes (17,18),
said device having a longitudinal axis (100) and a sagittal midplane (π) passing through said longitudinal axis (100),
said device comprising:
- an interlaminar proximal support portion (20) configured to be inserted between said pairs of right and left laminae (15a,b; 16a,b) and providing lower (23') and upper (23") support surfaces configured to abut against said pairs of laminae (15a,b; 16a,b) of said lower (10') and upper (10") vertebrae, respectively;
- a distal stabilization portion (30) adjacent to said interlaminar proximal support portion (20) along said longitudinal axis (100);
wherein said distal stabilization portion (30) comprises a tail element (36) extending from a side opposite to said interlaminar proximal support portion (20) and configured to abut against a root portion (17') of said spinous process (17) of said lower vertebra (10'),
wherein said distal stabilization portion (30) comprises an engagement section (32) for engaging with said laminae (15a,b; 16a,b), said engagement section comprising:
- a lower pair of right and left radial ramifications (33a,b) extending from a central core (31) of said stabilization portion (30) along respective lower radial axes (101a,b) and configured to abut against external faces (19) of said right and left laminae (15a,b), respectively, of said lower vertebra (10'), when said interlaminar proximal support portion (20) is inserted between said pairs of right and left laminae (15a,b; 16a,b);
- an upper pair of right and left radial ramifications (34a,b) extending from said central core (31) of said stabilization portion (30) along respective upper radial axes (102a,b) and configured to abut against external faces (19) of said right and left laminae (16a,b), respectively, of said upper vertebra (10"), when said interlaminar proximal support portion (20) is inserted between said pairs of right and left laminae (15a,b; 16a,b),
wherein said central core (31) has, between said upper pair of right and left radial ramifications (34a,b), an upper face (35) having a downward slope with respect to said support surfaces (23',23"), starting from a distal side (30') of said stabilization portion (30), adjacent to said interlaminar proximal support portion (20), and going towards a proximal end side (30") of said support device (2,3,5,6),
wherein said right (33a,34a) and left (33b,34b) radial ramifications of at least one pair selected between said lower pair (33a,33b) and said upper pair (34a,34b) are configured to resiliently perform respective rotations (ω₁, ω₂) about said longitudinal axis (100) from a rest position to a rotated position.

2. The device (2,3) according to claim 1, wherein
- said lower radial axes (101a,b) are at a predetermined lower inclination angle (α₁) with respect to said sagittal plane (π), said lower inclination angle set between 60° and 80°, in particular, between 65° and 75°;
- said upper radial axes (101a,b) are at a predetermined upper inclination angle (α₂) with respect to said sagittal plane (π), said upper inclination angle set between 45° and 60°°, in particular, between 50° and 55°.

3. The device (3) according to claim 1, wherein said right (33a,34a) and left (33b,34b) radial ramifications of both said lower and upper pairs are configured to resiliently perform said respective rotations (ω₁, ω₂).

4. The device according to claims 2, wherein said lower radial axes (101a,b) and said upper radial axes (102a,b) are at said lower and upper inclination angles (α₁, α₂), respectively, with respect to said sagittal plane (π) when said right (33a,34a) and left (33b,34b) radial ramifications are in said rest configuration (A).

5. The device (3) according to claim 1, wherein said right (33a,34a) and left (33b,34b) radial ramifications, which are configured to resiliently perform respective rotations (ω₁, ω₂), have at least a root portion that is made of a resiliently deformable material.

6. The device (5) according to claim 1, wherein said lower radial ramifications (33a,b) have a convex proximal face (38') and said upper radial ramifications (34a,b) have a concave proximal face (38").

7. The device (6) according to claim 1, wherein said support surfaces (23',23") of said interlaminar proximal support portion (20) have side parts (24) that extend more forward in a proximal direction than the respective central parts (25).

8. The device according to claim 1, wherein said tail element (36) has a protruding length (L₃), with respect to said right and left radial ramifications, that is shorter than 10 mm.

9. The device according to claim 8, wherein said protruding length (L3), with respect to said right and left radial ramifications, is shorter than 8 mm.

10. The device according to claim 1, wherein said tail element (36) has a lowered upper surface portion (37) forming a recess configured to contain the spinous process (18) of said upper vertebra (10").

11. The device (2,3,5) according to claim 1, wherein a distance (W1) between free end portions of right (33a,34a) and left (33b,34b) radial ramifications of said upper pair or of said lower pair of radial ramifications is set between 25 and 35 mm.

12. The device (2,3,5) according to claim 1, wherein a distance (H1) between the free end portions of said right radial ramifications (33a,34a) and between the free end portions of said left radial ramifications (33b,34b) is set between 14 and 22 mm.

13. The device (2,3,5) according to claim 1, wherein a protruding length (L2) of said upper support surface (23") of said interlaminar proximal support portion (20) from a proximal face (38") of said radial ramifications is set between 4 mm and 6 mm.

14. The device (2,3,5) according to claim 1, wherein a distance (H2) between said lower support surface (23') and said upper support surface (23") is set between 4 mm and 14 mm.

15. The device (3) according to claim 12, wherein said distance (H1) between the free end portions of said right and left radial ramifications is a rest distance in a rest configuration (A) and, in a resiliently-rotated configuration (B), the distance (H') between the free end portions of said right and left radial ramifications is shorter than 18 mm.

## Patentansprüche

1. Interlaminare Zwischenwirbelstützvorrichtung (2,3,5,6), welche derart konfiguriert ist, dass sie zwischen einem unteren Wirbel (10') und einem oberen Wirbel (10") implantiert werden kann, wobei die unteren und oberen Wirbel jeweils Paare rechter und linker Laminae (15a,b; 16a,b) und jeweils Dornfortsätze (17,18) aufweisen,
wobei die Vorrichtung eine Längsachse (100) und eine durch die Längsachse (100) verlaufende sagittale Mittelebene (π) aufweist,
wobei die Vorrichtung umfasst:
- einen interlaminaren proximalen Stützabschnitt (20), welcher derart konfiguriert ist, dass er zwischen die Paare rechter und linker Laminae (15a,b; 16a,b) eingebracht werden kann, und welcher untere (23') und obere (23") Stützoberflächen bereitstellt, die derart konfiguriert sind, dass sie an den Paaren von Laminae (15a,b; 16a,b) des unteren (10') bzw. oberen (10") Wirbels anliegen;
- einen distalen Stabilisierungsabschnitt (30), welcher entlang der Längsachse (100) an den interlaminaren proximalen Stützabschnitt (20) angrenzt;
wobei der distale Stabilisierungsabschnitt (30) ein Schwanzelement (36) umfasst, welches sich von einer dem interlaminaren proximalen Stützabschnitt (20) gegenüberliegenden Seite erstreckt und welches derart konfiguriert ist, dass es an einem Wurzelabschnitt (17') des Dornfortsatzes (17) des unteren Wirbels (10') anliegt,
wobei der distale Stabilisierungsabschnitt (30) einen Eingriffsbereich (32) zum Eingreifen in die Laminae (15a,b; 16a,b) umfasst, wobei der Eingriffsbereich umfasst:
- ein unteres Paar rechter und linker radialer Verzweigungen (33a,b), welche sich ausgehend von einem zentralen Kern (31) des Stabilisierungsabschnitts (30) entlang jeweiliger unterer Radialachsen (101a,b) erstrecken und welche derart konfiguriert sind, dass sie an äußere Flächen (19) der rechten bzw. linken Laminae (15a,b) des unteren Wirbels (10') anliegen, wenn der interlaminare proximale Stützabschnitt (20) zwischen die Paare rechter und linker Laminae (15a,b; 16a,b) eingebracht worden ist;
- ein oberes Paar rechter und linker radialer Verzweigungen (34a,b), welche sich ausgehend von dem zentralen Kern (31) des Stabilisierungsabschnitts (30) entlang jeweiliger oberer Radialachsen (102a,b) erstrecken und welche derart konfiguriert sind, dass sie an äußere Flächen (19) der rechten bzw. linken Laminae (16a,b) des oberen Wirbels (10") anliegen, wenn der interlaminare proximale Stützabschnitt (20) zwischen die Paare rechter und linker Laminae (15a,b; 16a,b) eingebracht worden ist,
wobei der zentrale Kern (31) zwischen dem oberen Paar rechter und linker radialer Verzweigungen (34a,b) eine in Bezug auf die Stützoberflächen (23',23") ein Gefälle aufweisende obere Fläche (35) aufweist, welche an einer distalen Seite (30') des Stabilisierungsabschnitts (30) neben dem interlaminaren proximalen Stützabschnitt (20) beginnt und in Richtung einer proximalen Endseite (30") der Stützvorrichtung (2,3,5,6) verläuft,
wobei die rechten (33a,34a) und linken (33b,34b) radialen Verzweigungen zumindest eines Paares ausgewählt aus dem unteren Paar (33a,33b) und dem oberen Paar (34a,34b) derart konfiguriert sind, dass sie aus einer Ruheposition heraus jeweils Drehungen (ω₁,ω₂) um die Längsachse (100) in eine gedrehte Position elastisch ausführen.

2. Vorrichtung (2, 3) gemäß Anspruch 1, wobei
- die unteren Radialachsen (101a,b) in Bezug auf die Sagittalebene (π) einen vorbestimmten unteren Neigungswinkel (α₁) aufweisen, wobei der untere Neigungswinkel auf zwischen 60° und 80°, insbesondere zwischen 65° und 75°, eingestellt ist;
- die oberen Radialachsen (101a,b) in Bezug auf die Sagittalebene (π) einen vorbestimmten oberen Neigungswinkel (α₂) aufweisen, wobei der obere Neigungswinkel auf zwischen 45° und 60°, insbesondere zwischen 50° und 55°, eingestellt ist.

3. Vorrichtung (3) gemäß Anspruch 1, wobei die rechten (33a,34a) und linken (33b,34b) radialen Verzweigungen sowohl der unteren als auch der oberen Paare derart konfiguriert sind, dass sie die jeweiligen Drehungen (ω₁,ω₂) elastisch ausführen.

4. Vorrichtung gemäß Anspruch 2, wobei die unteren Radialachsen (101a,b) und die oberen Radialachsen (102a,b) in Bezug auf die Sagittalebene (π) den unteren bzw. oberen Neigungswinkel (α₁,α₂) aufweisen, wenn sich die rechten (33a,34a) und linken (33b,34b) radialen Verzweigungen in der Ruhekonfiguration (A) befinden.

5. Vorrichtung (3) gemäß Anspruch 1, wobei die rechten (33a,34a) und linken (33b,34b) radialen Verzweigungen, welche derart konfiguriert sind, dass sie die jeweiligen Drehungen (ω₁,ω₂) elastisch ausführen, zumindest einen Wurzelabschnitt aufweisen, der aus einem elastisch verformbaren Material besteht.

6. Vorrichtung (5) gemäß Anspruch 1, wobei die unteren radialen Verzweigungen (33a,b) eine konvexe proximale Fläche (38') und die oberen radialen Verzweigungen (34a,b) eine konkave proximale Fläche (38") aufweisen.

7. Vorrichtung (6) gemäß Anspruch 1, wobei die Stützoberflächen (23',23") des interlaminaren proximalen Stützabschnitts (20) Seitenteile (24) aufweisen, welche sich in einer proximalen Richtung weiter nach vorne erstrecken als die jeweiligen Mittelteile (25).

8. Vorrichtung gemäß Anspruch 1, wobei das Schwanzelement (36) in Bezug auf die rechten und linken radialen Verzweigungen eine Überstandslänge (L3) aufweist, welche weniger als 10 mm beträgt.

9. Vorrichtung gemäß Anspruch 8, wobei die Überstandslänge (L3) in Bezug auf die rechten und linken radialen Verzweigungen weniger als 8 mm beträgt.

10. Vorrichtung gemäß Anspruch 1, wobei das Schwanzelement (36) einen abgesenkten oberen Oberflächenabschnitt (37) aufweist, welcher eine Aussparung bildet, die derart konfiguriert ist, dass sie den Dornfortsatz (18) des oberen Wirbels (10") enthält.

11. Vorrichtung (2,3,5) gemäß Anspruch 1, wobei ein Abstand (W1) zwischen freien Endabschnitten der rechten (33a,34a) und linken (33b,34b) radialen Verzweigungen des oberen Paares oder des unteren Paares radialer Verzweigungen auf zwischen 25 und 35 mm eingestellt ist.

12. Vorrichtung (2,3,5) gemäß Anspruch 1, wobei ein Abstand (H1) zwischen den freien Endabschnitten der rechten radialen Verzweigungen (33a,34a) und zwischen den freien Endabschnitten der linken radialen Verzweigungen (33b,34b) auf zwischen 14 und 22 mm eingestellt ist.

13. Vorrichtung (2,3,5) gemäß Anspruch 1, wobei eine Überstandslänge (L2) der oberen Stützoberfläche (23") des interlaminaren proximalen Stützabschnitts (20) von einer proximalen Fläche (38") der radialen Verzweigungen auf zwischen 4 mm und 6 mm eingestellt ist.

14. Vorrichtung (2,3,5) gemäß Anspruch 1, wobei ein Abstand (H2) zwischen der unteren Stützoberfläche (23') und der oberen Stützoberfläche (23") auf zwischen 4 mm und 14 mm eingestellt ist.

15. Vorrichtung (3) gemäß Anspruch 12, wobei der Abstand (H1) zwischen den freien Endabschnitten der rechten und linken radialen Verzweigungen einen Ruheabstand in einer Ruhekonfiguration (A) darstellt, und der Abstand (H') zwischen den freien Endabschnitten der rechten und linken radialen Verzweigungen in einer elastisch-gedrehten Konfiguration (B) weniger als 18 mm beträgt.

## Revendications

1. Dispositif de support intervertébral de type interlaminaire (2, 3, 5, 6) conçu pour être implanté entre une vertèbre inférieure (10') et une vertèbre supérieure (10"), lesdites vertèbres inférieure et supérieure possédant des paires de lames droite et gauche respectives (15a,b ; 16a,b) et des processus épineux respectifs (17, 18), ledit dispositif possédant un axe longitudinal (100) et un plan médian sagittal (π) passant par ledit axe longitudinal (100), ledit dispositif comprenant :
une partie de support proximale interlaminaire (20) conçue pour être insérée entre lesdites paires de lames droite et gauche (15a,b ; 16a,b) et fournissant des surfaces de support inférieure (23') et supérieure (23") conçues pour venir en butée contre lesdites paires de lames (15a,b ; 16a,b) desdites vertèbres inférieure (10') et supérieure (10"), respectivement ;
une partie de stabilisation distale (30) adjacente à ladite partie de support proximale interlaminaire (20) le long dudit axe longitudinal (100) ; ladite partie de stabilisation distale (30) comprenant un élément queue (36) s'étendant à partir d'un côté opposé à ladite partie de support proximal interlaminaire (20) et conçu pour venir en butée contre une partie racine (17') dudit processus épineux (17) de ladite vertèbre inférieure (10'),
ladite partie de stabilisation distale (30) comprenant une section de mise en prise (32) destinée à se mettre en prise avec lesdites lames (15a,b ; 16a,b), ladite section de mise en prise comprenant :
une paire inférieure de ramifications radiales droite et gauche (33a,b) s'étendant à partir d'un noyau central (31) de ladite partie de stabilisation (30) le long d'axes radiaux inférieurs respectifs (101a,b) et conçues pour venir en butée contre des faces externes (19) desdites lames droite et gauche (15a,b), respectivement, de ladite vertèbre inférieure (10'), lorsque ladite partie de support proximale interlaminaire (20) est insérée entre lesdites paires de lames droite et gauche (15a,b ; 16a,b) ;
une paire supérieure de ramifications radiales droite et gauche (34a,b) s'étendant à partir dudit noyau central (31) de ladite partie de stabilisation (30) le long d'axes radiaux supérieurs respectifs (102a,b) et conçues pour venir en butée contre des faces externes (19) desdites lames droite et gauche (16a,b), respectivement, de ladite vertèbre supérieure (10"), lorsque ladite partie de support proximale interlaminaire (20) est insérée entre lesdites paires de lames droite et gauche (15a,b ; 16a,b),
ledit noyau central (31) possédant, entre ladite paire supérieure de ramifications radiales droite et gauche (34a,b), une face supérieure (35) possédant une pente vers le bas par rapport auxdites surfaces de support (23', 23"), commençant à partir d'un côté distal (30') de ladite partie de stabilisation (30), adjacent à ladite partie de support proximale interlaminaire (20), et allant vers un côté d'extrémité proximale (30") dudit dispositif de support (2, 3, 5, 6),
lesdites ramifications radiales droite (33a, 34a) et gauche (33b, 34b) d'au moins une paire sélectionnée entre ladite paire inférieure (33a, 33b) et ladite paire supérieure (34a, 34b) étant conçues pour réaliser de manière élastique des rotations respectives (ω₁, (ω₂) autour dudit axe longitudinal (100) d'une position de repos à une position tournée.

2. Dispositif (2, 3) selon la revendication 1,
lesdits axes radiaux inférieurs (101a,b) étant à un angle d'inclinaison inférieur prédéfini (ai) par rapport audit plan sagittal (π), ledit angle d'inclinaison inférieur étant réglé entre 60° et 80°, en particulier, entre 65° et 75° ;
lesdits axes radiaux supérieurs (101a,b) étant à un angle d'inclinaison supérieur prédéfini (α₂) par rapport audit plan sagittal (π), ledit angle d'inclinaison supérieur étant réglé entre 45° et 60°, en particulier, entre 50° et 55°.

3. Dispositif (3) selon la revendication 1, lesdites ramifications radiales droite (33a, 34a) et gauche (33b, 34b) desdites deux paires inférieure et supérieure étant conçues pour réaliser de manière élastique lesdites rotations respectives (ω₁, ω₂).

4. Dispositif selon les revendications 2, lesdits axes radiaux inférieurs (101a,b) et lesdits axes radiaux supérieurs (102a,b) étant auxdits angles d'inclinaison inférieur et supérieur (α₁, α₂), respectivement, par rapport audit plan sagittal (π) lorsque lesdites ramifications radiales droite (33a, 34a) et gauche (33b, 34b) sont dans ladite configuration de repos (A).

5. Dispositif (3) selon la revendication 1, lesdites ramifications radiales droite (33a, 34a) et gauche (33b, 34b), qui sont conçues pour réaliser de manière élastique des rotations respectives (ω₁, ω₂), possédant au moins une partie racine qui est constituée d'un matériau élastiquement déformable.

6. Dispositif (5) selon la revendication 1, lesdites ramifications radiales inférieures (33a,b) possédant une face proximale convexe (38') et lesdites ramifications radiales supérieures (34a,b) possédant une face proximale concave (38").

7. Dispositif (6) selon la revendication 1, lesdites surfaces de support (23', 23") de ladite partie de support proximale interlaminaire (20) possédant des parties latérales (24) qui s'étendent plus vers l'avant dans une direction proximale que les parties centrales respectives (25).

8. Dispositif selon la revendication 1, ledit élément queue (36) possédant une longueur faisant saillie (L₃), par rapport auxdites ramifications radiales droite et gauche, qui est inférieure à 10 mm.

9. Dispositif selon la revendication 8, ladite longueur faisant saillie (L3), par rapport auxdites ramifications radiales droite et gauche, étant inférieure à 8 mm.

10. Dispositif selon la revendication 1, ledit élément queue (36) possédant une partie de surface supérieure abaissée (37) formant un évidement conçu pour contenir le processus épineux (18) de ladite vertèbre supérieure (10").

11. Dispositif (2, 3, 5) selon la revendication 1, une distance (W1) entre des parties d'extrémité libres des ramifications radiales droite (33a, 34a) et gauche (33b, 34b) de ladite paire supérieure ou de ladite paire inférieure des ramifications radiales étant réglée entre 25 et 35 mm.

12. Dispositif (2, 3, 5) selon la revendication 1, une distance (H1) entre les parties d'extrémité libres desdites ramifications radiales droites (33a, 34a) et entre les parties d'extrémité libres desdites ramifications radiales gauches (33b, 34b) étant réglée entre 14 et 22 mm.

13. Dispositif (2, 3, 5) selon la revendication 1, une longueur faisant saillie (L2) de ladite surface de support supérieure (23") de ladite partie de support proximale interlaminaire (20) à partir d'une face proximale (38") desdites ramifications radiales étant réglée entre 4 mm et 6 mm.

14. Dispositif (2, 3, 5) selon la revendication 1, une distance (H2) entre ladite surface de support inférieure (23') et ladite surface de support supérieure (23") étant réglée entre 4 mm et 14 mm.

15. Dispositif (3) selon la revendication 12, ladite distance (H1) entre les parties d'extrémité libre desdites ramifications radiales droite et gauche étant une distance de repos dans une configuration de repos (A) et, dans une configuration de rotation élastique (B), ladite distance (H') entre les portions d'extrémité libre desdites ramifications radiales droite et gauche étant inférieure à 18 mm.
